(19) Europäisches Patentamt
European Patent Office
Office européen des brevets

(11) **EP 3 285 815 B1**

(12) **EUROPÄISCHE PATENTSCHRIFT**

(45) Veröffentlichungstag und Bekanntmachung des Hinweises auf die Patenterteilung:
**26.02.2025  Patentblatt 2025/09**

(21) Anmeldenummer: **16710915.6**

(22) Anmeldetag: **08.03.2016**

(51) Internationale Patentklassifikation (IPC):
***A61L 2/08*** *(2006.01)*      ***B01D 15/20*** *(2006.01)*

(52) Gemeinsame Patentklassifikation (CPC):
**A61L 2/081; B01D 15/20; B01D 15/206**

(86) Internationale Anmeldenummer:
**PCT/EP2016/000405**

(87) Internationale Veröffentlichungsnummer:
**WO 2016/169630 (27.10.2016 Gazette 2016/43)**

(54) **VERFAHREN ZUR STERILISIERUNG EINES CHROMATOGRAPHIEMATERIALS UND DANACH STERILISIERTES CHROMATOGRAPHIEMATERIAL**

METHOD FOR THE STERILIZATION OF CHROMATOGRAPHIC MATERIAL AND CHROMATOGRAPHIC MATERIAL STERILIZED ACCORDING TO SAID METHOD

PROCÉDÉ DE STÉRILISATION DE MATÉRIEL DE CHROMATOGRAPHIE ET MATÉRIEL DE CHROMATOGRAPHIE STÉRILISÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.04.2015  DE 102015005244**

(43) Veröffentlichungstag der Anmeldung:
**28.02.2018  Patentblatt 2018/09**

(73) Patentinhaber: **Sartorius Stedim Biotech GmbH 37079 Göttingen (DE)**

(72) Erfinder:
• **LEUTHOLD, Martin 37079 Göttingen (DE)**
• **VILLAIN, Louis 37079 Göttingen (DE)**
• **TAFT, Florian 37079 Göttingen (DE)**

(74) Vertreter: **Novagraaf International SA Chemin de l'Echo 3 1213 Onex, Geneva (CH)**

(56) Entgegenhaltungen:
WO-A1-03/030949        WO-A1-2004/009143
WO-A1-2011/076386     WO-A1-2015/109146
WO-A1-2015/109246

**Beschreibung**

Die vorliegende Erfindung betrifft ein Verfahren zur Sterilisierung eines Chromatographiematerials, das eine mikroporöse Polymermembran ist.

Für Anwendungen in der biopharmazeutischen Industrie sind nicht nur sterile Endprodukte für die Verabreichung von Interesse, sondern auch sterile Materialien, welche bei der Herstellung von pharmazeutischen Wirkstoffen zum Einsatz kommen. Als Materialien werden hierbei Komponenten der einzelnen Prozessschritte sowie Medien bezeichnet. So sind zum Beispiel bei der Herstellung monoklonaler Antikörper schon während des Fermentationsschrittes ein steriles Fermentationsbehältnis und sterile Medien obligatorisch, um überhaupt ein entsprechendes Wachstum der Zelllinien zu gewährleisten. Nach Zellwachstum und Proteinbiosynthese muss der Antikörper in möglichst reiner Form von Zellen und anderen unerwünschten Komponenten getrennt werden. Hierbei kommen verschiedenste Prozessschritte zum Einsatz. Nach der Zellentfernung durch Tiefenfiltration oder Zentrifugation zählen dazu im Allgemeinen unterschiedlichste Chromatographieschritte zur Aufkonzentrierung und Reinigung des Zielmoleküls, Verfahren zur Virusentfernung sowie Konzentrierungs- und Sterilfiltrationsschritte. Auch während dieser Prozessschritte ist keimfreies Arbeiten vorteilhaft. Das Kontaminationsrisiko des finalen Produktes kann dadurch entsprechend minimiert werden.

Ein besonderer Fall stellt die Aufreinigung größerer Moleküle wie zum Beispiel Viren oder virusähnlichen Partikeln dar: Oftmals ist hier keine Sterilfiltration in der Aufarbeitung möglich. Durch die Größe des Moleküls würden eingesetzte Sterilfilter mit ihrer Porengröße das Produkt zurückhalten und eine Filtration unmöglich machen. Gerade hier sind sterile Prozesslösungen eine entscheidende Verbesserung.

Als Standard wird in vielen der erwähnten Prozessschritte das Risiko einer Kontamination durch Spülen mit Lauge oder den Einsatz von Hitze herabgesetzt. Diese Verfahren haben jedoch verschiedene Nachteile: oftmals ist die Umsetzung im Prozess komplex. Große Mengen an Lauge oder auch Heißdampf müssen zur Verfügung gestellt werden. Vor dem eigentlichen Prozessschritt muss eine zeitaufwendige Reinigung durchgeführt werden. Ein weiterer Nachteil ist oftmals die Stabilität der eingesetzten Materialien in den einzelnen Prozessschritten. So lässt sich zum Beispiel Heißdampf nicht einsetzen, um Chromatographiemedien zu sterilisieren. Andere Materialien weisen keine hinreichende Stabilität gegenüber Lauge auf.

In den letzten Jahren hat sich der Trend zum Einsatz von "Single-use"-Produkten bei der Herstellung von biopharmazeutischen Wirkstoffen verstärkt. Durch die Einmalanwendung und überwiegende Verwendung von Kunststoffen für Materialien wie Filtrationsvorrichtungen wurde damit auch der Weg zu alternativen Sterilisationsmethoden geebnet. Neben Autoklavieren werden Sterilisationsverfahren wie die Begasung mit Ethylenoxid oder Gammabestrahlung eingesetzt. Ein weiterer Vorteil ergibt sich hier für den Anwender auch in der Verfügbarkeit von vorsterilisierten Produkten für den Herstellprozess. Somit lassen sich Reinigungsaufwände für den Anwender reduzieren beziehungsweise entfallen komplett.

Im Allgemeinen können Sterilisationsmethoden in chemische und physikalische Verfahren unterteilt werden. Bei chemischen Verfahren ist der Einsatz von keimabtötenden Gasen weit verbreitet. Bei dieser Methode muss jedoch sichergestellt werden, dass alle Bereiche des Materials während der Sterilisation hinreichend mit dem Gas in Kontakt treten. Physikalische Methoden sind thermische Sterilisation und Bestrahlung. Die sterilisierende Wirkung der Bestrahlung beruht dabei auf der Zerstörung organischen Materials durch Bindungsspaltung. Vorteil einer Bestrahlung besteht darin, dass alle Bereiche eines Materials durchdrungen werden können.

Chromatographiemedien werden üblicherweise sanitisiert. Als Sanitisierung wird unter anderen die keimmindernde Behandlung mit Lauge bezeichnet. Häufig wird dabei eine bis zu 1-molare Lösung verwendet. Das Ergebnis dieser Behandlung ist dabei jedoch kein steriles Produkt beziehungsweise kein steriler Prozessschritt. Ein großer Nachteil dieser Behandlungsmethode ist weiterhin die Bereitstellung. Entweder muss der Aufwand zur Sanitisierung direkt vor der Verwendung im Prozess erfolgen, oder der Hersteller des chromatographischen Mediums muss das Medium bereits bei der Produktion in geeigneten Chromatographievorrichtungen sanitisieren und im nassen Zustand ausliefern. Dadurch erschwert sich nicht nur die Handhabung, es müssen auch aufwendige Studien zur Stabilität in der Lagerlösung durchgeführt werden. Vorsanitisierte Produkte in der Chromatographie sind als "Ready-to-use"-Produkte bekannt.

Zur Sterilisation kann auch die Autoklavierung eingesetzt werden. Dabei muss das Chromatographiemedium auf mindestens 121°C erwärmt werden. Für größere Maßstäbe ist dieses Verfahren jedoch ungeeignet, da für die Vorrichtungen aufgrund der Größe eine Umsetzung nicht möglich ist.

Eine weitere beschriebene Methode ist die Verwendung von Heißpuffern zur Sterilisation. Dabei wird das Chromatographiemedium ebenfalls auf mindestens 121°C erwärmt. Voraussetzung dafür ist, dass das Chromatographiesystem jedoch stabil gegenüber einer thermischen Belastung und Druck ist. Dieses Verfahren ist für Einwegsysteme ungeeignet.

US 2013/0062267 A1 beschreibt eine Sterilisationsmethode für die Bereitstellung von aseptischen Chromatographiesäulen. Dabei müssen das Medium und die Filtrationsvorrichtung (Chromatographiegehäuse) jedoch separat sterilisiert werden und anschließend das Chromatographiemedium mit sterilisiertem Equipment in die Vorrichtung eingebracht werden. Weiterhin ist die Methode nur für Polymergele anwendbar.

Haben sich im Bereich der Filtration und Fermentation bereits sterile Einweg-Fermentationsbehältnisse und Filtrationsvorrichtungen etabliert, so haben sich in der chromatographischen Anwendung bisher noch keine fertig verfügbaren sterilisierten Produkte etablieren können. Eine Hauptursache dafür ist die Stabilität der Materialien.

Dabei können sowohl Trägermatrix als auch Ligand durch Einwirkung von Hitze, Gammabestrahlung oder anderen Sterilisationsmethoden beschädigt werden oder sind nur unzureichend erreichbar. Als Trägermatrizes werden in der Chromatographie beispielsweise Polymergele, Membranen, Vliese, Folien oder Fasern eingesetzt. Liganden können unter anderem sein: Proteine, Substrate, Carboxymethyl, Sulfonate, quaternäre Amine, Aminoethyl, Metallchelate oder hydrophobe Gruppen. Die Bestrahlung der Materialien kann dabei beispielsweise die Seitenketten von Polymeren schädigen oder ablösen. Eine Behandlung mit oxidativen Medien wie mit reaktionsfähigem Ethylenoxid kann ebenso zu einer Änderung der Liganden führen. Bei den im Stand der Technik bekannten Sterilisierungsverfahren kommt es nachweislich zu einer Veränderung der Materialeigenschaften, wie beispielsweise direkt an Verfärbungen des Chromatographiematerials ersichtlich. Derartige Verfärbungen müssen zwar nicht zwangsläufig negativ hinsichtlich der Produktkontamination im Prozess sein, können nichtsdestotrotz wie beispielsweise in US 2011/0064608 A1 beschrieben aber unerwünscht sein.

WO 2004/009143 A1 beschreibt ein Verfahren zur Sterilisierung eines biologischen Materials. WO 2015/109146 A1 beschreibt eine sterile Chromatographie und Herstellungsverfahren. WO 03/030949 A1 beschreibt Verfahren zur Sterilisierung von biologischen Materialien. WO 2015/109246 A1 beschreibt das Sterilisieren von Chromatographiesäulen. WO 2011/076386 A1 beschreibt Container für Chromatographiemedien.

Der vorliegenden Erfindung liegt daher die Aufgabe zugrunde, ein Verfahren zur Sterilisierung eines Chromatographiematerials, welches eine mikroporöse Polymermembran ist, bereitzustellen, wobei das beanspruchte Verfahren die Materialeigenschaften des Chromatographiematerials nicht verändert, so dass das Chromatographiematerial für eine sterile Chromatographie eingesetzt werden kann.

Diese Aufgabe wird durch die in den Ansprüchen gekennzeichneten Ausführungsformen der vorliegenden Erfindung gelöst.

Die Erfindung wird durch den Gegenstand des Anspruchs 1 definiert, also durch ein Verfahren zur Sterilisierung eines Chromatographiematerials bereitgestellt, umfassend die Schritte:

(a) Behandeln des Chromatographiematerials mit einer wässrigen, basisch gepufferten Salzlösung; und
(c) Bestrahlen des im Schritt (a) behandelten Chromatographiematerials mit Gammastrahlung;

wobei das Chromatographiematerial eine mikroporöse Polymermembran ist. Gemäß einer bevorzugten Ausführungsform umfasst das erfindungsgemäße Verfahren weiter den Schritt (b) zwischen den Schritten (a) und (c) und/oder den Schritt (d) nach dem Schritt (c):

(b) Trocknen des im Schritt (a) behandelten Chromatographiematerials;
(d) Trocknen des im Schritt (c) bestrahlten Chromatographiematerials,

wobei in Schritt (c) das im Schritt (b) getrocknete Chromatographiematerial mit Gammastrahlung bestrahlt wird, wenn Schritt (b) durchgeführt wird.

Unter dem Begriff "Chromatographiematerial" wird im Sinne der vorliegenden Erfindung jegliches Material verstanden, dass für chromatographische Verfahren als stationäre Phase verwendet werden kann. Beim chromatographischen Verfahren wird dabei ein Stoffgemisch bedingt durch Wechselwirkungen zwischen den Einzelbestandteilen des Stoffgemisches mit der stationären und der mobilen Phase aufgetrennt.

Das Chromatographiematerial im erfindungsgemäßen Verfahren ist eine mikroporöse Polymermembran. Das erfindungsgemäße Verfahren eignet sich insbesondere für mikroporöse Polymermembranen, deren äußere und/oder innere Oberfläche(n) durch Liganden, wie beispielsweise Proteine, Substrate, Carboxymethyl, Sulfonate, quaternäre Amine, Aminoethyl, Metallchelate oder hydrophobe Gruppen modifiziert wurde(n), da durch das erfindungsgemäße Verfahren das Chromatographiematerial sterilisiert werden kann, ohne dass sich die Materialeigenschaften in Bezug auf Trägermatrix und Ligand verändern.

Gemäß der vorliegenden Erfindung ist das zu sterilisierende Chromatographiematerial eine (oberflächenmodifizierte) mikroporöse Polymermembran. Erfindungsgemäß wird unter dem Begriff "mikroporöse Polymermembran" eine Polymermembran mit einer Porengröße von 0,01 $\mu$m bis 10 $\mu$m verstanden. Gemäß der vorliegenden Erfindung kann die Membran Cellulose, Cellulosederivate, Polyester, Polyethylen (PE), Polyamid, Polyethersulfon (PES), Polyvinylidendifluorid (PVDF), Polytetrafluorethylen (PTFE), Polypropylen (PP) und Polysulfon als strukturgebenden Bestandteil umfassen, wobei diese Materialien einzeln oder in entsprechender Kombination Anwendung finden können.

Mikroporöse Polymermembranen für die Verwendung in chromatographischen Anwendungen eignen sich insbesondere zum Einbau in Filtrationsvorrichtungen (Chromatographiegehäuse), indem beispielsweise wie in DE 197 11 168 A1 beschrieben aus mehreren seriell angeordneten Polymermembran-Lagen ein chromatographisches Bett ausgebildet wird. In derartigen Vorrichtungen, die auch als Membranadsorber bezeichnet werden, liegt mindestens eine Lage einer Polymermembran als Bahnmaterial vor, wobei die mindestens eine Polymermembran üblicherweise eine Dicke von 50 μm bis 350 μm aufweist. Üblicherweise weist die mindestens eine Polymermembran eine innere Porosität von 50% bis 90% auf.

Im Schritt (a) des erfindungsgemäßen Verfahrens wird das zu sterilisierende Chromatographiematerial mit einer wässrigen, basisch gepufferten Salzlösung behandelt, um das Material für den Bestrahlungsschritt (c) des erfindungsgemäßen Verfahrens zu konservieren. Das Chromatographiematerial kann dabei entweder sofort nach seiner Herstellung, d.h. nach Herstellung des Materials und einer gegebenenfalls erfolgten Oberflächenmodifizierung, bei der entsprechende Liganden an der Matrix gebunden werden, oder auch erst nach Lagerung behandelt werden. Der Begriff "Behandeln" unterliegt erfindungsgemäß keiner besonderen Einschränkung. Beispielsweise wird das zu stabilisierende Chromatographiematerial mit einer wässrigen, basisch gepufferten Salzlösung versetzt und dabei geschüttelt, beispielsweise für eine Dauer von ungefähr 10 Minuten, um ein vollständiges Benetzen bzw. einen vollständigen Austausch mit der in den Poren vorhandenen Lösung zu erreichen. Dabei kann die Behandlung gemäß der vorliegenden Erfindung bei Raum- bzw. Umgebungstemperatur erfolgen. Erfindungsgemäß wird das zu behandelnde Chromatographiematerial beispielsweise in die vorstehend genannte Lösung in einem geeigneten Gefäß gegeben, wobei dieses anschließend auf einen Schüttler gestellt wird, der mit 30 bis 150 U/min geschwenkt wird. Alternativ kann die Behandlung des Chromatographiematerials erfindungsgemäß auch durch Rühren der Lösung, in der sich das Chromatographiematerial befindet, erfolgen. In einer weiteren Ausführungsform kann das Chromatographiematerial gemäß der vorliegenden Erfindung auch durch eine Wanne hindurchgeführt werden, in der die Lösung gegebenenfalls umgewälzt wird.

Der Schritt (a) des erfindungsgemäßen Verfahrens kann sowohl wie vorstehend beschrieben mit einem isolierten Chromatographiematerial durchgeführt werden, d.h. das Chromatographiematerial befindet sich nicht in einem (Chromatographie)-Gehäuse, als auch mit einem Chromatographiematerial, das bereits in einem (Chromatographie)-Gehäuse eingebaut ist, sich also in einem eingebauten Zustand befindet. Dabei wird erfindungsgemäß entsprechend die wässrige, basisch gepufferte Salzlösung als Behandlungslösung so in das Gehäuse eingebracht, dass die vorstehend beschriebenen Behandlungsziele erreicht werden. Beispielsweise kann das Gehäuse mit dem eingebauten Chromatographiematerial von der Behandlungslösung durchströmt werden. Weitere Erläuterungen für den Fall, dass das Chromatographiematerial bereits in ein Gehäuse eingebaut ist, erfolgen nachstehend.

Die im Schritt (a) verwendete wässrige, basisch gepufferte Salzlösung unterliegt erfindungsgemäß keiner besonderen Einschränkung.

Pufferlösungen enthalten eine Mischung aus einer schwachen Säure und ihrer konjugierten bzw. korrespondierenden Base (bzw. des jeweiligen Salzes) oder einer schwachen Base und ihrer konjugierten bzw. korrespondierenden Säure. Auch Ampholyte (bifunktionale Moleküle) können als Puffer dienen. Der den pH-Wert bestimmende Faktor ist das Verhältnis bzw. das Protolyse-Gleichgewicht des Pufferpaares. Gemäß der vorliegenden Erfindung sind grundsätzlich solche Säure-Base-Paare geeignet, die einen pKa von größer 4 aufweisen.

Die Salz- bzw. Pufferlösung gemäß der vorliegenden Erfindung kann ein oder mehrere Salze umfassen, solange die Pufferlösung einen pH-Wert größer 7, bevorzugt größer 8 aufweist.

Erfindungsgemäß wird Wasser als Lösungsmittel für die basisch gepufferte Salzlösung eingesetzt werden. Daneben sind auch Gemische möglich, wie beispielsweise eine Mischung aus Wasser und Glycerin. Grundsätzlich sind Lösungsmittel geeignet, mit denen sich gemäß der vorliegenden Erfindung entsprechende Salz- bzw. Pufferlösungen herstellen lassen, während das zu behandelnde Chromatographiematerial gegenüber solchen Lösungsmitteln inert ist. Gemäß einer bevorzugten Ausführungsform umfasst die Salzlösung im Schritt (a) mindestens ein Salz, ausgewählt aus der Gruppe, bestehend aus Natriumacetat, Natriumdihydrogenphosphat, Dinatriumhydrogenphosphat, Trinatriumphosphat, Natriumdihydrogencitrat, Dinatriumhydrogencitrat und Trinatriumcitrat. Gemäß einer bevorzugten Ausführungsform der vorliegenden Erfindung umfasst die Salzlösung in Schritt (a) Dinatriumhydrogenphosphat. Die Salzkonzentration der Salzlösung im Schritt (a) beträgt vorzugsweise von 0,01 bis 1 mol/L, besonders bevorzugt von 0,02 bis 0,1 mol/L. Bei einer Salzkonzentration von unter 0,01 mol/L kann bei der anschließenden Bestrahlung nicht ausgeschlossen werden, dass es aufgrund der Wirkung der Strahlung nicht doch zu einer Veränderung des behandelten Chromatographiematerials kommt. Bei einer Salzkonzentration von über 1 mol/L kann zudem nicht ausgeschlossen werden, dass das Chromatographiematerial nicht schon durch die Behandlungslösung selbst beschädigt wird bzw. einer ungewünschten Veränderung unterliegt.

Gemäß einer bevorzugten Ausführungsform umfasst die wässrige, basisch gepufferte Salzlösung im Schritt (a) weiter 10 bis 40 Gew.-% Glycerin. Dies ist insbesondere vorteilhaft, wenn das Chromatographiematerial wie gemäß der vorliegenden Erfindung eine poröse Polymermembran ist, da in diesem Fall das Glycerin die feine poröse Membranstruktur während der Trocknungsschritte (b) und (d) schützt.

Im Schritt (c) des erfindungsgemäßen Verfahrens wird das im Schritt (a) behandelte Chromatographiematerial mit Gammastrahlung bestrahlt, um das Chromatographiematerial zu sterilisieren. Vorteilhafterweise verändern sich die Materialeigenschaften des Chromatographiematerials aufgrund des erfolgten Konservierungsschritts (a) des erfindungsgemäßen Verfahrens durch die Gammabestrahlung nicht, wie dies bei Bestrahlung von im Stand der Technik bekannten unbehandelten Chromatographiematerialien, d.h. bei Materialien, die keinen Konservierungsschritt (a) erfahren haben, der Fall ist. Die Bestrahlung erfolgt gemäß der vorliegenden Erfindung beispielsweise unter Verwendung einer Cobaltquelle oder mit Elektronenstrahlen mit einer Energie von 1 bis 12 MeV. Dabei wird das zu bestrahlende Material beispielsweise auf Förderbändern um die Strahlenquelle befördert. Die Anzahl der Durchläufe hängt dabei von der gewünschten Minimaldosis ab, welche üblicherweise überwacht wird. Eine solche Bestrahlung kann erfindungsgemäß in Raumluft oder unter Schutzatmosphäre durchgeführt werden. Gemäß einer bevorzugten Ausführungsform wird im Schritt (c) mit einer Gammastrahlendosis von 1kGy bis 100 kGy bestrahlt. Bei einer Strahlendosis von weniger als 1kGy besteht die Gefahr, dass das Chromatographiematerial nicht ausreichend sterilisiert wird. Bei einer Strahlendosis von mehr als 100 kGy können die bestrahlten Materialien geschädigt werden, insbesondere können sie ihre Festigkeit verlieren und spröde werden. Besonders bevorzugt wird im Schritt (c) mit einer Strahlendosis von 15 kGy bis 85 kGy, insbesondere von 30 kGy bis 70 kGy, bestrahlt.

Wie schon vorstehend ausgeführt, umfasst das erfindungsgemäße Verfahren weiter bevorzugt den Schritt (b) zwischen den Schritten (a) und (c) und/oder den Schritt (d) nach dem Schritt (c):

(b) Trocknen des im Schritt (a) behandelten Chromatographiematerials;

(d) Trocknen des im Schritt (c) bestrahlten Chromatographiematerials,

wobei in Schritt (c) das im Schritt (b) getrocknete Chromatographiematerial mit Gammastrahlung bestrahlt wird, wenn Schritt (b) durchgeführt wird.

Im Schritt (b) und/oder im Schritt (d) des erfindungsgemäßen Verfahrens wird das im Schritt (a) behandelte Chromatographiematerial bzw. das im Schritt (c) bestrahlte Chromatographiematerial getrocknet. Der Begriff "Trocknen" unterliegt erfindungsgemäß keiner besonderen Einschränkung. Beispielsweise kann das Trocknen im Schritt (b) und/oder im Schritt (d) bei Raumtemperatur oder auch bei erhöhter Temperatur, beispielsweise in einem Ofen, erfolgen. Zusätzlich oder stattdessen kann das Trocknen gemäß der vorliegenden Erfindung durch Aufblasen oder Durchblasen trockener inerter Gase auf bzw. durch die in Schritt (a) behandelten Chromatographiematerialien und/oder die in Schritt (c) bestrahlten Chromatographiematerialien erfolgen. Gemäß einer bevorzugten Ausführungsform der vorliegenden Erfindung wird das Chromatographiematerial im Schritt (b) und/oder im Schritt (d) bei einer Temperatur von 70°C bis 90°C für eine Dauer von 5 min bis 20 min getrocknet.

Wenn die wässrige, basisch gepufferte Salzlösung wie vorstehend beschrieben in einer bevorzugten Ausführungsform Glycerin umfasst, kann dieses nach dem Trocknen eine Restfeuchte verursachen. Diese kann zum einen erfindungsgemäß im behandelten Chromatographiematerial verbleiben. Zum anderen kann gemäß der vorliegenden Erfindung durch die Durchführung sowohl des Schritts (b) als auch des Schritts (d) eine solche Restfeuchte weiter reduziert bzw. entfernt werden.

Gemäß einer Ausführungsform des erfindungsgemäßen Verfahrens wird das Chromatographiematerial nach dem Schritt (a) bzw. nach dem Schritt (b) in eine sterile, bestrahlungsfähige Verpackung eingeschlossen und dann in dieser Verpackung mit Gammastrahlung bestrahlt. Dadurch kann das durch Gammabestrahlung sterilisierte Chromatographiematerial vorteilhafterweise steril gelagert und transportiert werden, ohne dass die Gefahr einer nachträglichen Kontamination nach Bestrahlung besteht. Derartige sterile, bestrahlungsfähige Verpackungen sind dem Fachmann bekannt und beispielsweise unter dem registrierten Markennamen Tyvek® der Firma DuPont kommerziell erhältlich.

Beispielsweise wird ein Endprodukt, beispielsweise als geschlossenes System mit Sterilkonnektoren, in eine entsprechende Verpackung wie beispielsweise Beutel und Kartonage eingebracht und anschließend so verpackt bestrahlt. Dabei können gemäß der vorliegenden Erfindung sowohl Einzelkartonagen als auch Anordnungen dieser auf Paletten einer Bestrahlung unterliegen.

Gemäß einer weiteren Ausführungsform der vorliegenden Erfindung befindet sich das Chromatographiematerial zumindest bei Durchführung des Schritts (c) des Verfahrens bereits eingebaut in einem Gehäuse. Dabei kann das Gehäuse gemäß einer bevorzugten Ausführungsform erfindungsgemäß bereits in eine bestrahlungsfähige Verpackung eingebracht sein.

Zudem kann das Chromatographiematerial erfindungsgemäß auch während der Durchführung des gesamten Verfahrens, d.h. bei Durchführung der Schritte (a) (wie schon vorstehend beschrieben), gegebenenfalls (b), (c) und gegebenenfalls (d) bereits in ein Gehäuse eingebaut sein. Entweder ist dabei für eine Endanwendung des Chromatographiematerials eine Trocknung desselben nicht notwendig, oder die Ausführungsform des Gehäuses lässt die Schritte (b) und/oder (d) in Form von beispielsweise Anwendung von Wärme und/oder des Aufblasens oder Durchblasens entsprechender Gase zu.

Des Weiteren wird hier ein Chromatographiematerial beschrieben, das durch das erfindungsgemäße Verfahren sterilisiert wurde. Das erfindungsgemäß sterilisierte Chromatographiematerial kann vorteilhafterweise zur chromatographischen Entfernungen von Kontaminanten aus einem Fluid unter sterilen Bedingungen verwendet werden. Derartige chromatographische Entfernungsverfahren sind dem Fachmann bekannt.

Weiter wird hier ein geschlossenes, chromatisches Filtrationssystem beschrieben, umfassend eine sterile Filtrationsvorrichtung, die ein erfindungsgemäß sterilisiertes Chromatographiematerial umfasst, assemblierte sterile Schläuche und Sterilverbindungen. Derartige Filtrationssysteme ohne das erfindungsgemäß sterilisierte Chromatographiematerial sind dem Fachmann bekannt.

Das Verfahren der vorliegenden Erfindung ermöglicht es vorteilhafterweise, ein Chromatographiematerial, das eine (oberflächenmodifizierte) mikroporöse Polymermembran ist, zu sterilisieren, ohne dass sich die Materialeigenschaften des Chromatographiematerials verändern. Das erfindungsgemäß sterilisierte Chromatographiematerial kann nach Sterilisierung vorteilhafterweise gelagert werden, ohne sich zu verfärben. Da sich die Adsorptionskapazität des Chromatographiematerials durch die erfindungsgemäße Sterilisierung vorteilhafterweise nicht verändert, kann das erfindungsgemäß sterilisierte Chromatographiematerial insbesondere für die chromatographische Entfernung von Kontaminanten aus einem Fluid unter sterilen Bedingungen eingesetzt werden.

Die vorliegende Erfindung wird anhand der folgenden, nicht einschränkenden Beispiele näher erläutert.

## Beispiele

Vergleichsbeispiel:

Mit Wasser benetzte Membranadsorber vom Typ 1 bis 3 wurden mit einer wässrigen Glycerin-Lösung mit einem Anteil von 30 Gew.-% Glycerin unter Schütteln für 10 min bei Raumtemperatur behandelt und anschließend für 10 Minuten bei 80°C im Trockenschrank getrocknet.

Typ 1: MA Sartobind® S der Sartorius Stedim Biotech GmbH, kommerziell verfügbar

Typ 2: MA Sartobind® HIC Phenyl der Sartorius Stedim Biotech GmbH, kommerziell verfügbar

Typ 3: Membran, hergestellt nach DE 10 2013 017 014 der Sartorius Stedim Biotech GmbH

Erfindungsgemäßes Sterilisierungsverfahren:

Die mit Wasser benetzten Membranadsorber vom Typ 1 bis 3 wurden wie im Vergleichsbeispiel behandelt, wobei die Glycerin-Lösung mit einem Anteil von 30 Gew.-% Glycerin zusätzlich 1 Gew.-% eines Salzes (Natriumacetat-Trihydrat oder Dinatriumhydrogenphosphat-Dihydrat oder Trinatriumcitrat-Trihydrat) enthielt.

Ausführungsbeispiel 1: Verfärbung bei der Sterilisation von Membranadsorbern

Zur Bestimmung der Verfärbung nach Gammabestrahlung mit einer Dosis von mindestens 50 kGy (minimal gemessene Oberflächendosis 50,7 kGy, maximal gemessene Oberflächendosis 55,8 kGy) wurden rechteckige Membranadsorberzuschnitte einer Stabilitätsstudie unterzogen. Dazu wurden Membranzuschnitte von Membranen, die mit einer wässrigen, basisch gepufferten Salzlösung (Bezeichnungen in den Figuren: "Acetat", "Phosphat", "Natriumcitrat") behandelt wurden, und von Membranen, die nicht dem erfindungsgemäßen Behandlungsschritt ausgesetzt wurden (Bezeichnung in den Figuren: "Standard") nach der Bestrahlung unter kontrollierten Bedingungen (40°C ± 2°C, 75% ± 5% rel. Luftfeuchtigkeit) beschleunigt gealtert. Erfahrungsgemäß entspricht eine Lagerung pro 100 Tage unter den gewählten beschleunigten Alterungsbedingungen ca. 1,5 Jahre Lagerung unter Normalbedingungen. Unter den gleichen beschleunigten Alterungsbedingungen wurde auch ein Membranzuschnitt einer Membran gealtert, die zunächst unter Einsatz von Ethylenoxid sterilisiert wurde (Bezeichnung in den Figuren: "EtO"), d.h. erst mit Ethylenoxid behandelt wurde und anschließend der vorstehend beschriebenen Gammabestrahlung ausgesetzt wurde.

Die Zuschnitte wurden nach der Spülung in gammastabile Kunststofftüten eingeschweißt und gelagert. Nach definierten Zeitabständen (0, 100, 200 Tage) wurden Proben der Membran entnommen und untersucht.

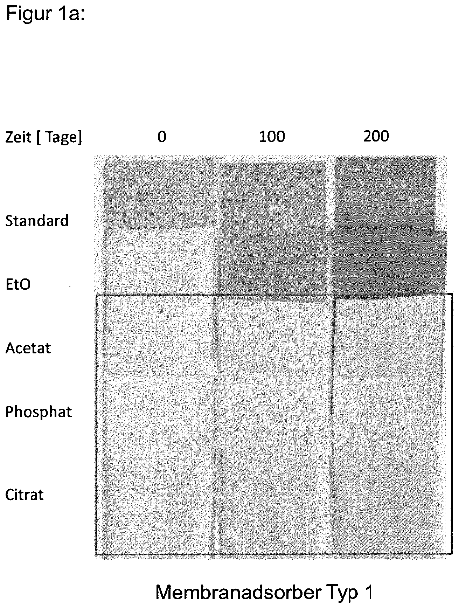

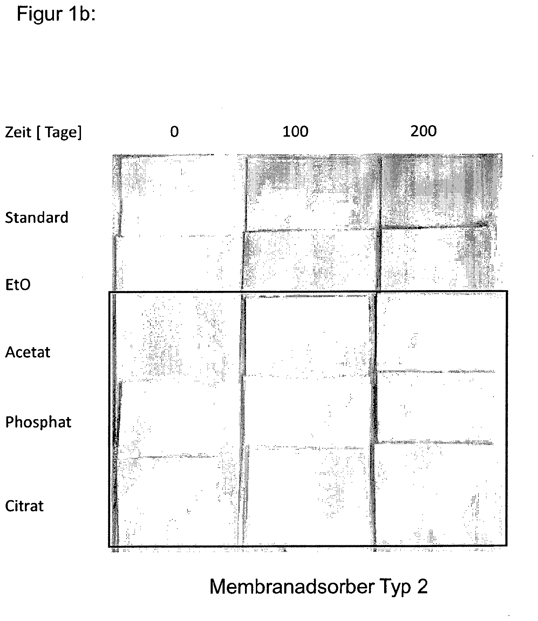

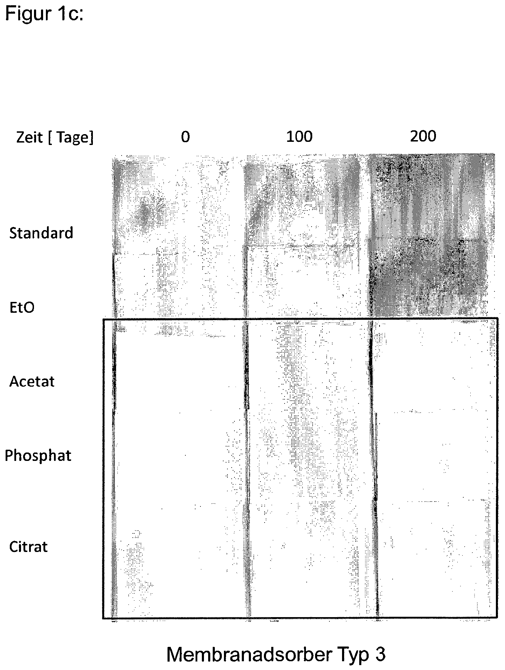

Figur 1 zeigt die optische Erscheinung der jeweiligen Membranen nach 0, 100 und 200 Tagen.

Die Alterungsstudie zeigt, dass über einen Zeitraum von mindestens 200 Tagen das optische Erscheinungsbild der erfindungsgemäß sterilisierten Membranadsorber im Gegensatz zu einem sterilisierten Membranadsorber ohne vorhergegangene Anwendung des erfindungsgemäßen Behandlungsschritts (a) vorteilhafterweise stabil bleibt, d.h. keine Veränderungen der Materialeigenschaften in Form von Verfärbungen aufgetreten sind.

Ausführungsbeispiel 2: Einfluss auf die Adsorptionskapazität

Mit dem erfindungsgemäßen Verfahren wurde eine Kationenaustauschermembran basierend auf stabilisierter Zellulose des Typs MA Sartobind® S der Sartorius Stedim Biotech GmbH, kommerziell verfügbar, unter den vorstehend aufgeführten Bedingungen (siehe "Erfindungsgemäßes Sterilisierungsverfahren" und "Ausführungsbeispiel 1") gespült und sterilisiert.

Anschließend wurde folgendermaßen die Bindekapazität ermittelt: 1 g/L Lysozym wurden in Phosphatpuffer (Pufferbestandteile Kaliumhydrogenphosphat und Dikaliumhydrogenphosphat) mit einer Molarität

11      **EP 3 285 815 B1**      12

von 10 mM und einem pH-Wert von 7,0 gelöst. Drei Lagen zu je 5 cm$^2$ des Membranadsorbers wurden in ein Filtrationsgehäuse eingebaut und an ein Chromatographiesystem angeschlossen (Äkta Explorer, GE Healthcare). Vor der Beladung mit der Proteinlösung wurde der Adsorber mit 20 mL eines 10 mM Phosphatpuffer pH 7 bei 10 mL/min equilibriert.

Die dynamische Kapazitätsbestimmung erfolgte durch eine Absorptionsmessung bei 280 nm mit einem UV-VIS Photometer an der Chromatographieanlage Äkta Explorer der GE Healthcare. Die Berechnung der Konzentration aus der gemessenen Extinktion erfolgte nach dem Lambert-Beerschen Gesetz. Der Extinktionskoeffizient für Lysozym wurde mit $2{,}47{*}10^{-3}$ L/(g*cm) bestimmt.

Die Bindekapazität wurde für den Zeitpunkt bestimmt, an dem 10% der Konzentration der Ausgangslösung am Ausgang des Filtrationsgehäuses gemessen wurde.

$$Kap_{10\%} = \frac{(V_{10\%} - V_{tot})*E(AL)}{\varepsilon * d * A_{Membran}}$$

$Kap_{10\%}$:    Dynamische Kapazität 10% [mg/cm$^2$]

$V_{10\%}$:    Beladenes Volumen der Ausgangslösung bei Durchbruch 10% der Konzentration der Ausgangslösung [L]

$V_{tot}$:    Totvolumen des Chromatographiesystems [L]

$E(AL)$:    Extinktion der Ausgangslösung

$\varepsilon$:    Spezifischer Extinktionskoeffizient = $2{,}47 * 10^{-3}$ [L/(mg*cm)]

$d$:    Schichtdicke UV-Messung = 1 [cm]

$A_{Membran}$:    Membranfläche [cm$^2$]

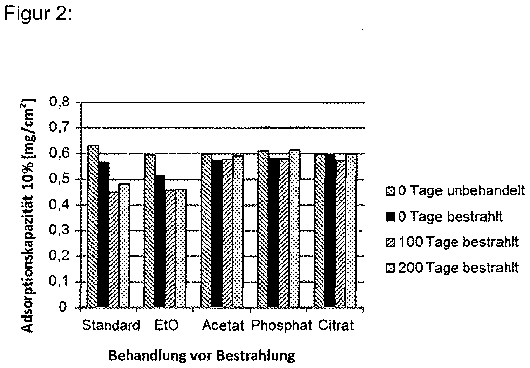

Figur 2 zeigt die Einwirkung der erfindungsgemäßen Verfahrensschritte auf die maßgeblichen Adsorptionseigenschaften der Membran.

Wie deutlich aus Figur 2 ersichtlich, beeinträchtigt vorteilhafterweise das erfindungsgemäße Verfahren (Bestrahlung mit einer Dosis von mindestens 50 kGy nach erfindungsgemäßem Spülschritt) die Adsorptionskapazität der Membran nicht signifikant im Vergleich zu nicht sterilisierten Membranadsorbern, d.h. das erfindungsgemäße Sterilisierungsverfahren hat vorteilhafterweise nicht die Materialeigenschaften des Chromatographiematerials verändert. Membranen, die ohne erfindungsgemäßem Spülschritt bestrahlt wurden, zeigen hingegen eine Abnahme der Adsorptionskapazität aufgrund von Änderungen der Materialeigenschaften.

Die Figuren zeigen:

Figur 1:    Optische Erscheinung von Membranen mit und ohne durchgeführtem erfindungsgemäßem Sterilisierungsverfahren nach definierten Alterungsdauern (0, 100 und 200 Tage).

Figur 2:    Vergleich der Adsorptionseigenschaften von Membranen mit und ohne durchgeführtem erfindungsgemäßen Sterilisierungsverfahren.

## Patentansprüche

1. Verfahren zur Sterilisierung eines Chromatographiematerials, umfassend die Schritte:

     (a) Behandeln des Chromatographiematerials mit einer wässrigen, basisch gepufferten Salzlösung; und
     (c) Bestrahlen des im Schritt (a) behandelten Chromatographiematerials mit Gammastrahlung;

wobei das Chromatographiematerial eine mikroporöse Polymermembran ist.

2. Verfahren nach Anspruch 1, weiter umfassend den Schritt (b) zwischen den Schritten (a) und (c) und/oder den Schritt (d) nach dem Schritt (c):

     (b) Trocknen des im Schritt (a) behandelten Chromatographiematerials;
     (d) Trocknen des im Schritt (c) bestrahlten Chromatographiematerials,

wobei in Schritt (c) das im Schritt (b) getrocknete Chromatographiematerial mit Gammastrahlung bestrahlt wird.

3. Verfahren nach Anspruch 1 oder 2, wobei die mikroporöse Polymermembran eine Dicke von 50 bis 350 μm aufweist.

4. Verfahren nach einem der Ansprüche 1 bis 3, wobei die wässrige, basisch gepufferte Salzlösung im Schritt (a) mindestens ein Salz, ausgewählt aus der Gruppe, bestehend aus Natriumacetat, Natriumdihydrogenphosphat, Dinatriumhydrogenphosphat, Trinatriumphosphat, Natriumdihydrogencitrat, Dinatriumhydrogencitrat und Trinatriumcitrat, umfasst.

5. Verfahren nach einem der Ansprüche 1 bis 4, wobei die Salzkonzentration der Salzlösung im Schritt (a) von 0,01 bis 1 mol/L beträgt.

6. Verfahren nach einem der Ansprüche 1 bis 5, wobei die wässrige, basisch gepufferte Salzlösung im Schritt (a) weiter 10 bis 40 Gew.-% Glycerin umfasst.

7. Verfahren nach einem der Ansprüche 2 bis 6, wobei das Chromatographiematerial im Schritt (b) und/oder im Schritt (d) in einem Temperaturbereich von 70°C bis 90°C für eine Dauer von 5 min bis 20 min getrocknet wird.

7

**8.** Verfahren nach einem der Ansprüche 1 bis 7, wobei im Schritt (c) mit einer Gammastrahlendosis von 1 kGy bis 100 kGy bestrahlt wird.

**9.** Verfahren nach einem der Ansprüche 1 bis 8, wobei sich das Chromatographiematerial bei Durchführung des Verfahrens eingebaut in einem Gehäuse befindet.

**10.** Verfahren nach einem der Ansprüche 1 bis 8, wobei sich das Chromatographiematerial im Schritt (c) in einer bestrahlungsfähigen Verpackung befindet.

**11.** Verfahren nach Anspruch 9, wobei sich das Gehäuse mit dem eingebauten Chromatographiematerial im Schritt (c) in einer bestrahlungsfähigen Verpackung befindet.

**Claims**

**1.** Method for sterilizing a chromatography material, comprising the steps:

(a) treating the chromatography material with an aqueous, alkaline-buffered salt solution; and
(c) irradiating the chromatography material treated in step (a) with gamma radiation;

wherein the chromatography material is a microporous polymer membrane.

**2.** Method according to claim 1, further comprising step (b) between steps (a) and (c) and/or step (d) after step (c):

(b) drying the chromatography material treated in step (a);
(d) drying the chromatography material irradiated in step (c) ;

wherein in step (c) the chromatography material dried in step (b) is irradiated with gamma radiation.

**3.** Method according to claim 1 or 2, wherein the microporous polymer membrane has a thickness of 50 to 350 $\mu$m.

**4.** Method according to any one of claims 1 to 3, wherein the aqueous, alkaline-buffered salt solution in step (a) comprises at least one salt selected from the group consisting of sodium acetate, sodium dihydrogen phosphate, disodium hydrogen phosphate, trisodium phosphate, sodium dihydrogen citrate, disodium hydrogen citrate, and trisodium citrate.

**5.** Method according to any one of claims 1 to 4, wherein the salt concentration of the salt solution in step (a) is from 0.01 to 1 mol/l.

**6.** Method according to any one of claims 1 to 5, wherein the aqueous, alkaline-buffered salt solution in step (a) further comprises 10 to 40 wt% glycerol.

**7.** Method according to any one of claims 2 to 6, wherein the chromatography material in step (b) and/or in step (d) is dried in a temperature range of 70°C to 90°C for a duration of 5 min to 20 min.

**8.** Method according to any one of claims 1 to 7, wherein in step (c) irradiation takes place using a gamma radiation dose of 1 kGy to 100 kGy.

**9.** Method according to any one of claims 1 to 8, wherein the chromatography material is situated in an integrated state in a housing when carrying out the method.

**10.** Method according to any one of claims 1 to 8, wherein the chromatography material in step (c) is situated in an irradiatable packaging.

**11.** Method according to claim 9, wherein the housing containing the integrated chromatography material in step (c) is situated in an irradiatable packaging.

**Revendications**

**1.** Procédé de stérilisation d'un matériel de chromatographie, comprenant les étapes de :

(a) traitement du matériel de chromatographie avec une solution aqueuse de sel tamponnée à l'alcalin ; et
(c) irradiation du matériel de chromatographie traité à l'étape (a) avec un rayonnement gamma ;

dans lequel le matériel de chromatographie est une membrane polymère microporeuse.

**2.** Procédé selon la revendication 1, comprenant en outre l'étape (b) entre les étapes (a) et (c) et/ou l'étape (d) après l'étape (c) :

(b) séchage du matériel de chromatographie traité à l'étape (a) ;
(d) séchage du matériel de chromatographie traité à l'étape (c),

dans lequel, à l'étape (c), le matériel de chromatographie séché à l'étape (b) est irradié avec un rayon gamma.

**3.** Procédé selon la revendication 1 ou 2, dans lequel la membrane polymère microporeuse présente une épaisseur de 50 à 350 μm.

4. Procédé selon l'une des revendications 1 à 3, dans lequel la solution aqueuse de sel tamponnée à l'alcalin comprend, à l'étape (a), au moins un sel, choisi dans le groupe constitué de l'acétate de sodium, du dihydrogénophosphate de sodium, de l'hydrogénophosphate de disodium, du phosphate de trisodium, du dihydrogénocitrate de sodium, de l'hydrogénocitrate de disodium et du citrate de trisodium.

5. Procédé selon l'une des revendications 1 à 4, dans lequel la concentration en sel de la solution de sel à l'étape (a) est de 0,01 à 1 mole/litre.

6. Procédé selon l'une des revendications 1 à 5, dans lequel la solution aqueuse de sel tamponnée à l'alcalin, à l'étape (a), comprend en outre 10 à 40 % en poids de glycérine.

7. Procédé selon l'une des revendications 2 à 6, dans lequel le matériel de chromatographie, à l'étape (b) et/ou à l'étape (d), est séché dans une plage de températures de 70 °C à 90 °C pendant une durée de 5 min à 20 min.

8. Procédé selon l'une des revendications 1 à 7, dans lequel, à l'étape (c), on irradie avec une dose de rayons gamma de 1 kGy à 100 kGy.

9. Procédé selon l'une des revendications 1 à 8, dans lequel le matériel de chromatographie se trouve intégré dans un boîtier lors de la réalisation du procédé.

10. Procédé selon l'une des revendications 1 à 8, dans lequel le matériel de chromatographie, à l'étape (c), se trouve dans un emballage pouvant supporter l'irradiation.

11. Procédé selon la revendication 9, dans lequel le boîtier avec le matériel de chromatographie intégré à l'étape (c) se trouve dans un emballage pouvant supporter l'irradiation.

## Figur 1a:

| Zeit [ Tage] | 0 | 100 | 200 |

Standard

EtO

Acetat

Phosphat

Citrat

Membranadsorber Typ 1

Figur 1b:

Membranadsorber Typ 2

Figur 1c:

Zeit [ Tage]       0          100          200

Standard

EtO

Acetat

Phosphat

Citrat

Membranadsorber Typ 3

## Figur 2:

**EP 3 285 815 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20130062267 A1
- US 20110064608 A1
- WO 2004009143 A1
- WO 2015109146 A1
- WO 03030949 A1
- WO 2015109246 A1
- WO 2011076386 A1
- DE 19711168 A1
- DE 102013017014